# EUROPEAN PATENT APPLICATION

(11) **EP 0 755 682 A1**
(43) Date of publication of application: **29.01.1997**
(21) Application number: 96111711.6
(22) Date of filing: 19.05.1992
(51) Int. Cl.: A61K 38/18

(54) **Methods of treatment of motor neuron diseases using members of the BDNF/NT-3/NGF family of molecules**

(30) Priority: 10.07.1991 US 727704
(62) Divisional of application: 92912453.5
(71) Applicant: REGENERON PHARMACEUTICALS, INC., Tarrytown, NY 10591-6707 (US); Max-Planck-Gesellschaft zur Förderung der Wissenschaften e.V., D-37073 Göttingen (DE)
(72) Inventor: Wong, Vivien, Scarsdale, NY 10583 (US); Radziejewski, Czeslaw, North White Plains, NY 10603 (US); Distefano, Peter, Carmel, NY 10512 (US)
(74) Representative: Goldin, Douglas Michael

(57) **Abstract**

This application relates to the use, in the manufacture of a medicament for the treatment of a motor neuron disorder, of
(a) Brain-Derived Neurotrophic Factor
   (BDNF); and
(b) ciliary neurotrophic factor (CNTF).

## Description

### 1. INTRODUCTION

The present invention relates to methods of treatment of motor neuron disorders comprising administering to a patient in need of such treatment an effective amount of a neurotrophic factor that is a member of the BDNF/NT-3/NGF family of molecules and that supports the survival, growth, and/or differentiation of motor neurons, preferably BDNF or NT-3. It also provides for methods of culturing motor neurons, methods of diagnosing disorders of motor neurons, assay systems for identifying agents which may be beneficial or toxic to motor neurons, and animal models for motor neuron diseases.

### 2. BACKGROUND OF THE INVENTION

### 2.1. MOTOR NEURONS

The interaction between motor neurons and muscle cells determines muscle tone and effects muscular contraction, and thereby underlies all voluntary and involuntary movement.

Motor neurons are traditionally classified as upper motor neurons or lower motor neurons. Upper motor neurons reside in the precentral gyrus of the brain, and send long processes down to synapse on lower motor neurons in the ventral (anterior) horns of the grey matter of the spinal cord. From the ventral horns of the spinal cord, axon processes of lower motor neurons coalesce to form the ventral roots. These axons eventually terminate on one or more muscle fibers. Through arborization of the terminal part of its fiber, each lower motor neuron comes in contact with anywhere from a few up to 100-200 or more muscle fibers to form a "motor unit," (Adams and Victor, 1985, in "Principles of Neurology," McGraw-Hill, Inc., New York, p. 37).

Motor neuron disorders result in varying degrees of muscle weakness, causing disability which ranges from slight difficulty performing difficult tasks to total paralysis. Lower motor neuron disorders are generally associated with a flaccid paralysis and decrease in muscle tone. Contiguous groups of muscles, innervated by single nerves or whose motor neurons lie close together in the spinal cord may be affected, and atrophy may be quite profound, up to 70-80 percent of total bulk. A diseased motor neuron may become irritable, and muscle fibers that it controls may discharge sporadically, in isolation from other units, to produce a visible twitch, or fasciculation. In contrast, when upper motor neurons are damaged, a spastic paralysis, with increase in muscle tone and hyperactive tendon reflexes, generally results. Usually, an entire limb or one-half of the body, rather than individual muscle groups, is affected. Atrophy is slight and typically results from lack of use. Fasciculations are absent. The identification of a clinical syndrome as representing upper or lower motor neuron damage may facilitate its diagnosis and management.

### 2.2. MOTOR NEURON DISORDERS

A wide array of neurological disorders may affect motor neurons. Upper motor neurons, for example, are predominantly affected by cerebrovascular accidents, neoplasms, infections and trauma. Lower motor neurons, or anterior horn cells, are secondarily affected by these processes, but in addition are subject to a number of disorders in which anterior horn cell loss is the primary feature, including amyotrophic lateral sclerosis, infantile and juvenile spinal muscular atrophy, poliomyelitis and the postpolio syndrome, hereditary motor and sensory neuropathies, and toxic motor neuropathies (e.g. vincristine).

Of these predominantly anterior horn cell (AHC) disorders, amyotrophic lateral sclerosis (ALS or Lou Gehrig's disease) is the most common (see Williams and Windebank, 1991, Mayo Clin. Proc. 66:54-82 for review).

The initial complaint in most patients with ALS is weakness, more commonly of the upper limbs (Gubbay et al., 1985, J. Neurol. 232:295-300; Vejjajiva et al., 1967, J. Neurol. Sci. 4:299-314; Li et al., 1988, J. Neurol. Neurosurg. Psychiatry 51:778-784). Usually the pattern of weakness, atrophy, and other neurological signs is asymmetric and often focal (Munsat et al., 1988, Neurol. 38:409-413). Muscle cramps, paresthesias (tingling sensations) and pain are frequent complaints (Williams and Windebank, supra). Widespread fasciculations are usually present (id.). The rate of progression of the disease varies from patient to patient (Gubbay et al., 1985, J. Neurol. 232:295-300), but in virtually all cases the disease eventually results in complete incapacity, widespread paralysis (including respiratory paralysis) and death.

Anatomically, the most prominent changes are atrophy of the spinal cord and associated ventral roots and firmness of the lateral columns (hence the name, amyotrophic lateral sclerosis; Williams and Windebank, supra). Upper motor neurons are also involved and degenerate in ALS. The brain may appear normal macroscopically, although atrophy of the motor and promotor cortices is usually present due to upper motor neuron involvement. There is widespread loss of Bets cells and other pyramidal cells from the precentral cortex, with consequent reactive gliosis (Hammer et al., 1979, Exp. Neurol. 63:336-346).

Epidemiologic studies have indicated that genetic as well as environmental factors play a role in disease development (Williams and Windebank, supra). Once believed to be a disease of middle-aged adults, it has recently been appreciated that ALS is more a disease of elderly persons (id.).

Current treatment consists of symptomatic therapy to diminish muscle cramps, pain, and fatiguability. Prosthetic devices are used to compensate for muscle weakness. Pharmacologic therapy to alter the progress of the disease has, however, been largely unsuccessful. Putative therapeutic benefits of thyrotropin releasing hormone have met with conflicting results (Brooks, 1989, Ann. N.Y. Acad. Sci. 553:431-461). Administration of gangliosides has been ineffective (Lacomblez et al., 1989, Neurol. 39:1635-1637). Plasmapheresis has shown no therapeutic advantage both alone and in combination with immunosuppressive treatment (Olarte et al., 1980, Ann. Neurol. 8:644-645; Kelemen et al., 1983, Arch. Neurol. 40:752-753). The antiviral agent guanidine was reported to have potential short-term benefits, but the results were not reproducible (Munsat et al., 1981, Neurol. 31:1054-1055). Administration of branched-chain amino acids to activate glutamate dehydrogenase was reported to slow the rate of decline of patients in an abbreviated study (Plaitakis et al., 1988, Lancet 1:1015-1018). Most recent therapeutic trials, some in progress, involve whole-body total lymphoid irradiation, the use of amino acids N-acetyl-cysteine, N-acetylmethionine, L-threonine, and longterm intrathecal infusion of thyrotropin releasing hormone (Williams and Windebank, supra).

Animal models that bear clinical and pathologic resemblances to ALS include the Mnd mouse, an autosomal dominant mutant exhibiting late-onset progressive degeneration of both upper and lower motor neurons (Messer and Flaherty, 1986, J. Neurogen. 3:345-355); the wobbler mouse, that exhibits forelimb weakness and atrophy in early life due to muscle denervation, and hereditary canine spinal muscular atrophy in the Brittany spaniel (Sack et al., 1984, Ann. Neurol. 15:369-373; Sillevis et al., 1989, J. Neurol. Sci. 91:231-258; Bird et al., 1971, Acta Neuropathol. 19:39-50).

### 2.3. CILIARY NEUROTROPHIC FACTOR AND MOTOR NEURON SURVIVAL

It has recently been shown in PCT Publication No. WO 91/04316, published April 4, 1991 and incorporated by reference herein that ciliary neurotrophic factor (CNTF) is capable of promoting the survival of motor neurons in vitro and in vivo. Gelfoam implants containing CNTF facilitated survival of motor neurons in severed facial nerves of newborn rats. CNTF is a neurotrophic factor that exhibits biological activities that are very different from those exhibited by the neurotrophin family of factors, including BDNF, NT-3, and NGF.

### 3. SUMMARY OF THE INVENTION

The present invention relates to methods of treatment of motor neuron disorders, such as amyotrophic lateral sclerosis (Lou Gehrig's disease) comprising administering, to a patient in need of such treatment, effective amounts of a neurotrophic factor that is a member of the BDNF/NT-3/NGF family of molecules and that supports the survival, growth and/or differentiation of motor neurons. It is based, in part, on the discovery that BDNF and NT-3 are each able to increase choline acetyltransferase activity in motor neuron cultures and are retrogradely transported from sciatic nerve to spinal cord. In preferred, specific embodiments of the invention, a combination of BDNF or NT-3 and a second agent, such as CNTF, may be used in the treatment of motor neuron disease.

The present invention also provides for methods of promoting the survival, growth and/or differentiation of motor neurons in vitro, comprising adding, to motor neuron cultures, an effective concentration of a member of the BDNF/NT-3/NGF gene family.

In additional embodiments, the present invention provides for assay systems which may be used to identify agents which have BDNF or NT-3-like activity and which may be useful in the treatment of motor neuron diseases.

The present invention further provides for animal models of motor neuron disease produced by immunizing animals to BDNF/NT-3/NGF family members or derivatives thereof or by administering to animals anti-BDNF/NT-3/NGF family member antibodies or antisense oligonucleotides to BDNF/NT-3/NGF.

### 3.1. ABBREVIATIONS

- BDNF: brain derived neurotrophic factor
- bFGF: basic fibroblast growth factor
- CAT: choline acetyltransferase
- NGF: nerve growth factor
- NT-3: neurotrophin-3

### 4. DESCRIPTION OF THE FIGURES

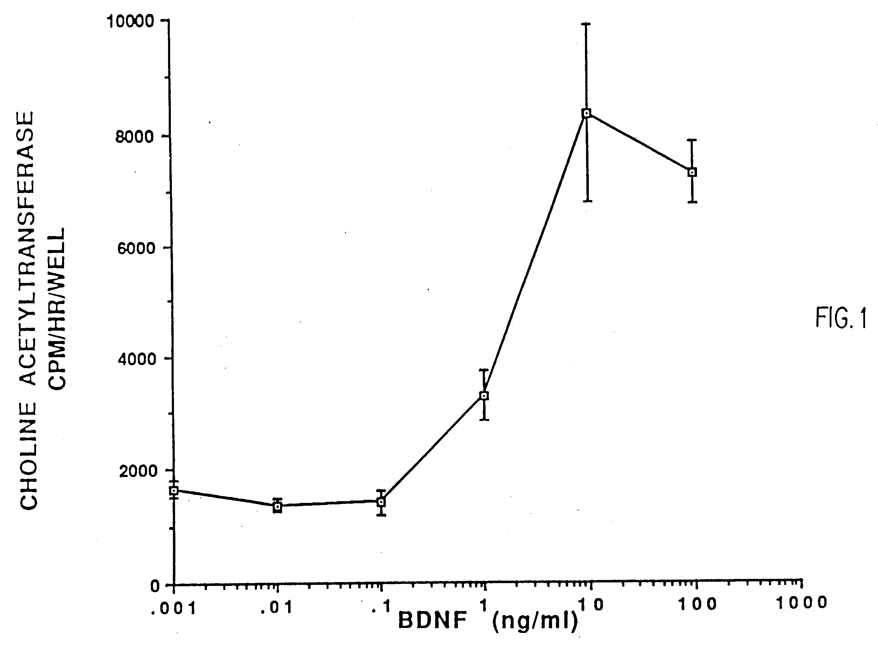

Figure 1. Motor neuron enriched cultures were grown in serum-free, chemically defined medium for two days and assayed for choline acetyltransferase activity (CPM/hr/well +/- SEM). BDNF was added at the time of plating at concentrations ranging from 10 pg/ml to 100 ng/ml using phosphate buffered saline (PBS)/0.5 mg/ml BSA as diluent. Control cultures received PBS/0.5 mg/ml BSA only.

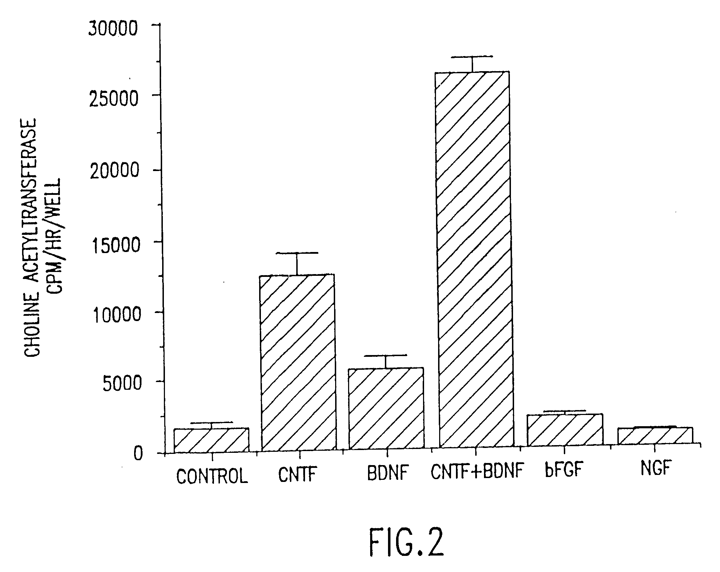

- Figure 2.: Motor neuron enriched cultures were grown in serum-free, chemically defined medium for two days and assayed for choline acetyl-transferase activity (CPM/hr/well +/- SEM). CNTF, BDNF, bFGF, NGF and CNTF/BDNF together (all at concentrations of 50 ng/ml) diluted in PBS/0.5 mg/ml BSA were added at the time of plating. Control cultures received PBS/0.5 mg/ml BSA.

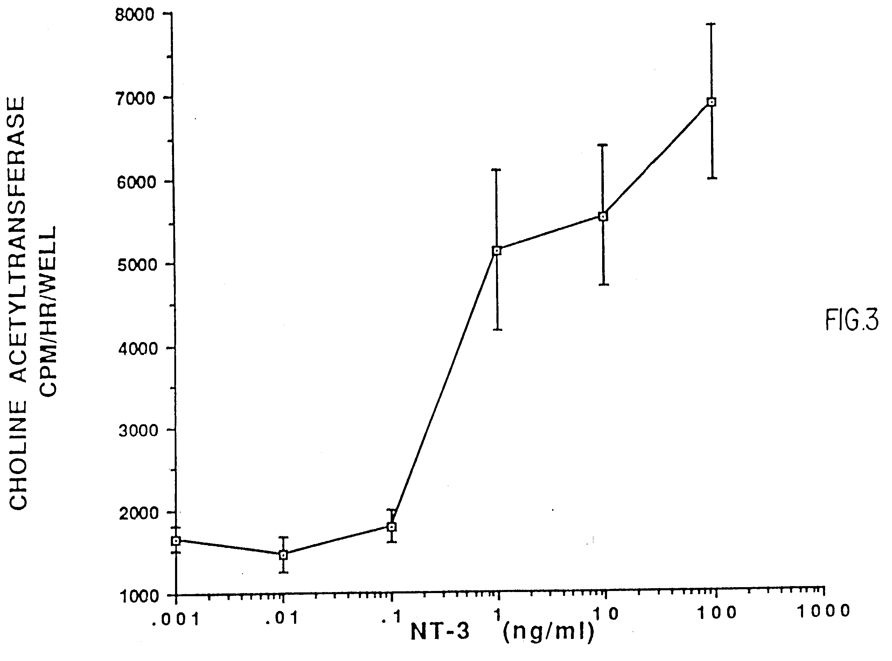

- Figure 3.: Motor neuron enriched cultures were grown in serum-free chemically defined medium for two days and assayed for choline acetyltransferase activity (CPM/hr/well +/- SEM). NT-3 was added at the time of plating at concentrations ranging from 10 pg/ml to 100 ng/ml.

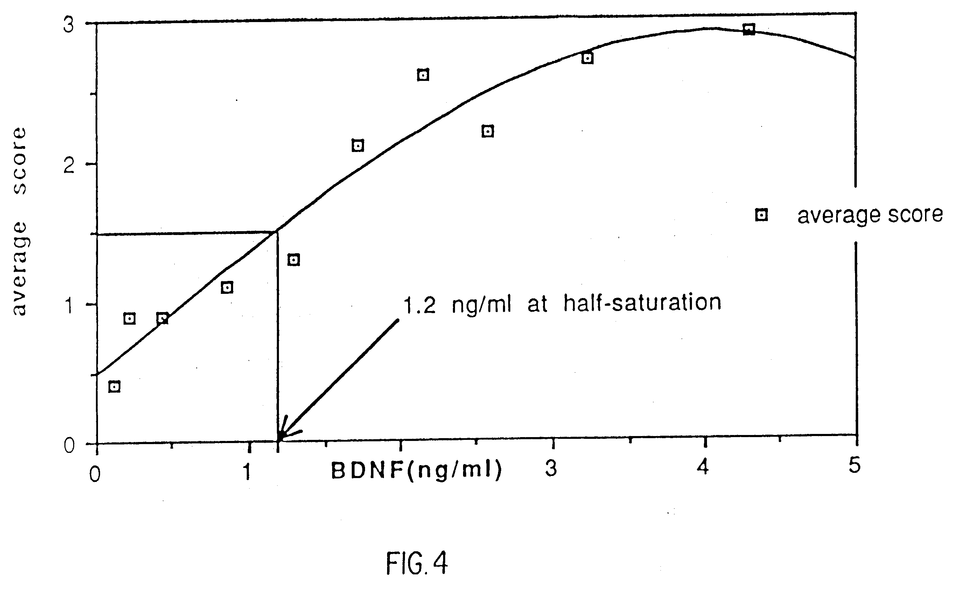

- Figure 4.: Dose-response curve for recombinant BDNF purified as described in Section 8 in dorsal root ganglion explant bioassay.

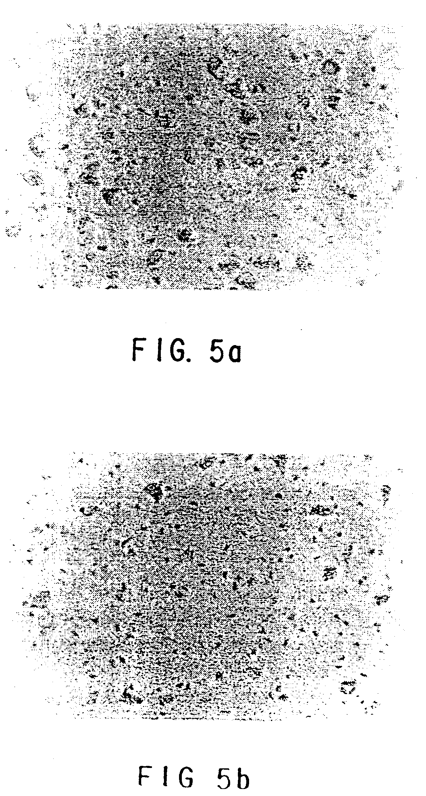

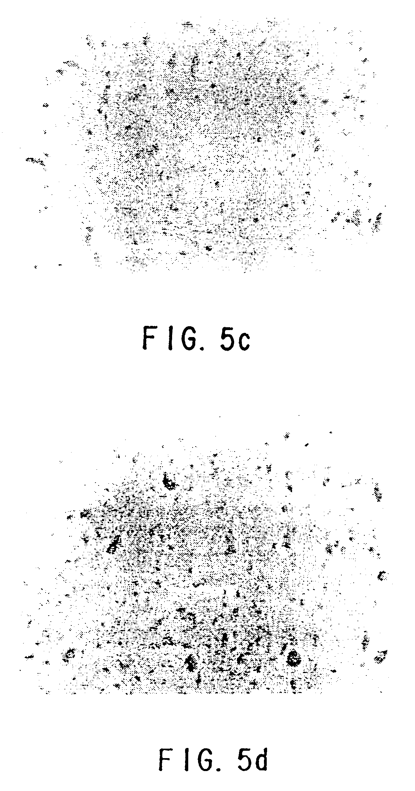

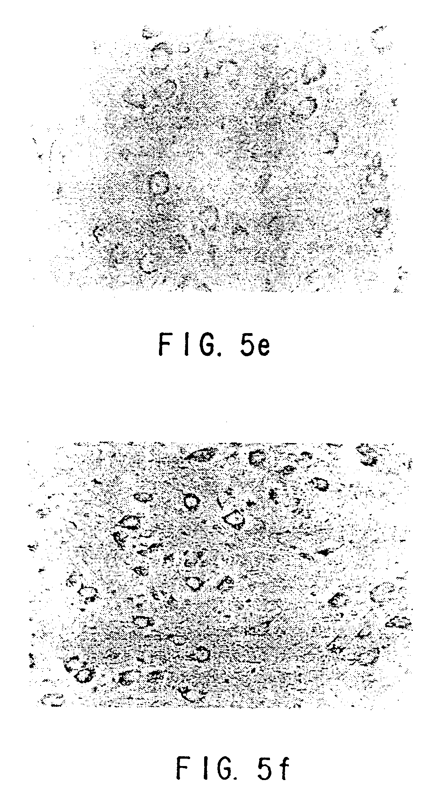

- Figure 5.: Morphology of facial motor neurons in the brainstem of 7 day old rats after unilateral facial nerve lesion at birth. a) lesioned side after BDNF treatment; b) lesioned side after NT-3 treatment; c) lesioned side after NGF-treatment; d) lesioned side after BSA treatment as a control; e) unlesioned contralateral side of the same animal as in a) control; f) after lesion and CNTF treatment as a control. Magnification: x400

### 5. DETAILED DESCRIPTION OF THE INVENTION

For purposes of clarity of disclosure, and not by way of limitation, the detailed description of the invention is divided into-thee following subsections:
(i) members of the BDNF/NT-3/NGF family of molecules that may be used according to the invention;
(ii) therapeutic applications;
(iii) in vitro applications;
(iv) diagnostic applications;
(v) assay systems; and
(vi) animal models.

### 5.1. MEMBERS OF THE BDNF/NT-3/NGF FAMILY OF MOLECULES THAT MAY BE USED ACCORDING TO THE INVENTION

It has been discovered that BDNF or NT-3 induce an increase in choline acetyltransferase activity in motor neuron cultures, indicating that certain members of the neurotrophin family may be used to promote survival, growth, and/or differentiation of motor neurons (see Example Sections 6 and 7, infra). That these neurotrophins act directly on motor neurons is supported by the observation that BDNF and NT-3 are retrogradely transported to ventral spinal cord, the situs of motor neurons (see Example Section 9, infra).

The present invention provides methods of promoting the survival, growth and/or differentiation of motor neurons which utilize members of the BDNF/NT-3/NGF family of molecules.

Preferred embodiments of the invention provide for use of members of the BDNF/NT-3/NGF family that result in an increase in choline acetyltransferase activity in motor neurons, relative to untreated motor neurons.

In the most preferred embodiments of the invention, BDNF or NT-3, as described in PCT Publication No. WO 91/03568 published March 21, 1991, and PCT Publication No. WO 91/03569 published March 21, 1991, incorporated by reference in their entireties herein, may be used to promote the survival and/or differentiation of motor neurons. As shown in example sections 6 and 7, infra, BDNF or NT-3 may be used to promote choline acetyltransferase activity in motor neurons in-culture. In addition, other members of the BDNF/NT-3/NGF family of molecules, which may be identified according to methods set forth in PCT Publication Nos.

WO 91/03568 and WO 91/03569 incorporated by reference in their entireties herein, may be used according to the invention provided that they are determined to be able to support the growth and/or differentiation of motor neurons. Furthermore, synthetic molecules, which are different in primary structure from any known member of the BDNF/NT-3/NGF family, and which support the growth and/or differentiation of motor neurons may be used. Because BDNF and NT-3 are more active than NGF in increasing choline acetyltransferase activity in motor neurons, such synthetic molecules may desirably resemble BDNF and NT-3 more than NGF.

The members of the BDNF/NT-3/NGF family, for use according to the invention, may be produced by any method known in the art. They may be purified from natural tissue, chemically synthesized, or, preferably, produced in recombinant DNA expression systems such as prokaryotic or eukaryotic expression systems, such as, for example, the COS cell system. Methods for producing these molecules include those described in PCT Publication Nos. WO 91/03568 and WO 91/03569.

In a preferred specific embodiment of the invention, BDNF or NT-3 may be isolated from medium conditioned by eukaryotic cells (e.g. BDNF transfected Chinese Hamster Ovary (CHO) cells)) expressing BDNF by a two step procedure consisting of cation exchange and gel filtration chromatography. Using BDNF as an example, conditioned medium collected from Opticell bioreactor (Charles River) seeded with cells expressing BDNF may be diluted with distilled water at 1.25:1 ratio and continuously loaded at onto a S-Sepharose Fast Flow (Pharmacia) column. For example, a total volume of 10.6L (diluted) may be applied over a period of about five days to a 15 ml (1.6 cm i.d.) column. The column may be washed with 50 ml of 20 mM MES buffer pH 5.8, followed by 50 ml of the same buffer containing 250 mM sodium chloride followed by the same MES buffer without sodium chloride until absorbance at 280 nm reached baseline level. The column may then be washed with 25 ml of 20 mM bicine buffer pH 8.5 followed by 15 ml of the same buffer containing 100 mM sodium chloride. The column may subsequently be eluted with 150 ml, 100 mM to 750 mM sodium chloride gradient in 20 mM bicine buffer pH 8.5 The flow rate may be about 2.5 ml/min. Absorbance of the eluate may be monitored at 280 nm at a sensitivity of 0.2 absorbance unit full scale. Fractions containing recombinant BDNF may be identified by DRG explant bioassay. The fractions with the highest specific activity may be expected to correspond to a sodium chloride concentration between 500 and 750 mM. These fractions may be combined and concentrated by high pressure membrane ultrafiltration using 3 KDa Omega type membrane, or an equivalent membrane that exhibits low nonspecific binding of BDNF. The concentrated sample may then be applied onto for example, a 120 ml (1.6 cm i.d.) Sephacryl S-100HR gel filtration column (Pharmacia). This column may be packed and equilibrated with 20 mM HEPES buffer pH 7.6 containing 400 mM NaCl. The column may be eluted at 0.25 ml/min. Fractions may be collected and assayed for BDNF activity in the DRG explant bioassay. Fractions eluted between 80 and 90 ml may be expected to contain bioactive recombinant brain derived growth factor. These fractions may be combined and analyzed by reducing SDS polyacrylamide electrophoresis, N-terminal sequencing and quantitative amino acid analysis. Volumes may be adjusted where appropriate.

The BDNF/NT-3/NGF family of molecules include those molecules encoded by genes with a region of substantial homology shared with BDNF, NT-3 and NGF. Members of the BDNF/NT-3/NGF family that are useful according to the invention may be selected by testing for biological activity in promoting the survival or differentiation of motor neurons. For example, and not by way of limitation, members of the BDNF/NT-3/NGF family which elicit any of the following effects may be useful according to the invention:
(i) increased survival time of motor neurons in culture;
(ii) increased sprouting of motor neurons in culture or in vivo;
(iii) increased production of a motor neuronassociated molecule in culture or in vivo, e.g. choline acetyltransferase or acetylcholinesterase (see Sections 6 and 7 infra for examples of measurement techniques); or
(iv) decreased symptoms of motor neuron dysfunction in vivo.

Such effects may be measured by any method known in the art. In preferred, non-limiting embodiments, increased survival of motor neurons may be measured by the method set forth in Arakawa et al., (1990, J. Neurosci. 10:3507-3515); increased sprouting of motor neurons may be detected by methods set forth in Pestronk et al. (1980, Exp. Neurol. 70:65-82) or Brown et al. (1981, Ann. Rev. Neurosci. 4:17-42); increased production of motor neuron-associated molecules may be measured by bioassay, enzymatic assay, antibody binding, Northern blot assay, etc., depending on the molecule to be measured; and motor neuron dysfunction may be measured by assessing the physical manifestation of motor neuron disorder, e.g. weakness, motor neuron conduction velocity, or functional disability.

In additional embodiments of the invention, the BDNF/NT-3/NGF family members of the invention may be combined with a second agent in order to support motor neuron survival and/or differentiation. Desirably, the second agent is also effective in promoting motor neuron survival and/or differentiation. For example, in a preferred embodiment of the invention, a BDNF/NT-3/NGF family member may be combined with CNTF in order to promote motor neuron survival and/or differentiation. As discussed in section 6, infra, BDNF combined with CNTF has been observed to exert at least an additive effect on motor neuron CAT activity.

### 5.2. THERAPEUTIC APPLICATIONS

The present invention provides for methods of treatment of a motor neuron disorder comprising administering, to a patient in need of such treatment, an effective amount of a neurotrophic factor that is a member of the BDNF/NT-3/NGF family of molecules and that supports the survival, growth or differentiation of motor neurons.

As used herein, the term motor neuron disorder refers to any condition that disturbs the normal function of motor neurons. Such disorders may or may not specifically or even selectively affect motor neurons. For example, motor neuron disorders that may be treated according to the invention include but are not limited to disorders such as infarction, infection, exposure to toxin, trauma, surgical damage, degenerative disease or malignancy that may affect motor neurons as well as other components of the nervous system. The methods of the invention may also be directed toward disorders that selectively affect neurons such as amyotrophic lateral sclerosis, and including, but not limited to, progressive spinal muscular atrophy, progressive bulbar palsy, primary lateral sclerosis, infantile and juvenile muscular atrophy, progressive bulbar paralysis of childhood (Fazio-Londe syndrome), poliomyelitis and the post polio syndrome, and Hereditary Motorsensory Neuropathy (Charcot-Marie-Tooth Disease).

Members of the BDNF/NT-3/NGF family which may be used according to the invention are described in section 5.1, supra. Effective doses may be determined using methods known to one skilled in the art. Effective doses and, if present, toxic doses (no toxic effect has as yet been identified for any known members of the BDNF/NT-3/NGF family may be determined. preferably in vitro, in order to identify the optimal dose range (see section 5.3, infra).

In various particular embodiments of the invention, neurotrophic factors of the BDNF/NT-3/NGF family may be administered together with an effective amount of ate least one other agent that is, itself, capable of promoting motor neuron survival, growth, or differentiation. For example, members of the BDNF/NT-3/NGF family may be administered together with CNTF. In a preferred, specific embodiment of the invention, BDNF and CNTF may be coadministered together in the treatment of a motor neuron disorder.

By referring to members of the BDNF/NT-3/NGF family, the present invention also contemplates the use of fragments, derivatives, agonists or antagonists of BDNF/NT-3/NGF family neurotrophic molecules.

The neurotrophic factor(s) of the invention may be administered in any pharmacologically acceptable carrier. The administration route may be any mode of administration known in the art, including but not limited to intravenously, intrathecally, subcutaneously, by injection into involved tissue, intraarterially, intranasally, orally, or via an implanted device. The present invention provides for pharmaceutical compositions comprising a member of the BDNF/NT-3/NGF family, such as BDNF or NT-3, together with CNTF in a pharmacologically acceptable carrier.

Administration may result in the distribution of the neurotrophic factor(s) of the invention throughout the body or in a localized area. For example, in some conditions which involve distant regions of the nervous system, intravenous or intrathecal administration of neurotrophic factor may be desirable. Alternatively, and not by way of limitation, when localized regions of the nervous system are involved, such as in motor neuron disorders caused by trauma or surgery, local administration may be desirable. In such situations, an implant containing neurotrophic factor may be placed in or near the lesioned area. Suitable implants include, but are not limited to, gelfoam, wax, or microparticle-based implants.

### 5.3. IN VITRO APPLICATIONS

The present invention provides for a method of promoting motor neuron survival, growth, and/or differentiation comprising exposing motor neurons to an effective concentration of a neurotrophic factor that is a member of the BDNF/NT-3/NGF family of molecules. This method may be carried out in vivo (see supra) or in vitro. In preferred embodiments, the BDNF/NT-3/NGF family member is BDNF or NT-3.

In in vitro embodiments, effective amounts of neurotrophic factor may desirably be determined on a case by case basis, as motor neurons from different tissue sources or from different species may exhibit different sensitivities to neurotrophic factor. For any particular culture, it may be desirable to construct a dose response curve that correlates neurotrophic factor concentration and motor neuron response. To evaluate motor neuron survival, growth, and/or differentiation, it may be useful to compare motor neurons exposed to a BDNF/NT-3/NGF family member neurotrophic factor to motor neurons not exposed to such factor, using, for example, vital dyes to evaluate survival, phase-contrast microscopy and/or neurofilament stain to measure neurite sprouting, or techniques that measure the bioactivity of motor neuron-associated compounds, such as choline acetyltransferase (CAT). CAT activity may be measured, for example, by harvesting and lysing treated and untreated motor neurons in a 20 mM Tris-HCl (pH 8.6) solution containing about 0.1% Triton X-100, removing an aliquot of several microliters, and measuring for CAT activity using, as a substrate, 0.2 ml (1 - ^{γ}C] acetyl-CoA, 300 mM NaCl, 8 mM choline bromide, 20 mM EDTA, and 0.1 mM neostigmine in 50 mM NaH₂ (pH 7.4) buffer, using the micro-Fonnum procedure as described in Fonnum, 1975, J. Neurochem. 24: 407-409, incorporated by reference in its entirety herein (see also section 7.1.5, infra).

In a specific, non-limiting embodiment of the invention, motor neurons may be prepared, and cultured in vitro, as follows. At least a portion of a spinal cord, preferably obtained from an embryonic organism such as a rat, may be aseptically obtained and separated from the bulb, sensory ganglia, and adhering meninges. The ventral segments of the cord may then be isolated, as motor neurons are localized in the ventral (anterior) horns of the spinal cord. Ventral cord segments may be diced into small pieces and incubated in about 0.1% trypsin and 0.01% deoxyribonuclease type 1 in calcium and magnesium-free phosphate buffered saline (PBS) at 37° for about 20 minutes. The trypsin solution may then be removed, and the cells may be rinsed and placed in fresh medium, such as 45% Eagle's minimum essential (MEM), 45% Ham's nutrient mixture F12, 5% heat inactivated fetal calf serum, 5% heat inactivated horse serum, glutamine (2 mM), penicillin G (0.5 U/ml), and streptomycin (0.5 µg/ml). The tissue may be mechanically dissociated by gentle trituration through a Pasteur pipet, and the supernatants pooled and filtered through a nylon filter (e.g. Nitex, Tetko; 40m). The filtered cell suspension may then be fractionated using a modification of the method set forth in Schnaar and Schaffner (1981, J. Neurosc. 1:204-217). All steps are desirably carried out at 4°C. Metrizamide may be dissolved in F12:MEM medium (1:1) and a discontinuous gradient may be established that consists of a 18% metrizamide cushion (e.g. 0.5 ml), 3 ml of 17% metrizamide; 3 ml of 12% metrizamide, and 3 ml of 8% metrizamide. The filtered cell suspension (e.g. 2.5 ml) may be layered over the steps gradient and the tube may be centrifuged at 2500 g for about 15 minutes using a swing-out rotor (e.g. Sorvall HB4). Centrifugation may be expected to result in three layers of cells: fraction I (at 0-8% interface), fraction II (at 8-12% interface) and fraction III (at 12-17% interface). Fraction I, enriched for motor neurons, may be removed in a small volume (e.g. about 1 ml) and rinsed twice with a serum-free defined medium such as 50% F12 and 50% MEM supplemented with glutamine (2 mM), insulin (5 µg/ml), transferrin (100 µg/ml), progesterone (20 nM), putrescine (100 µM), and sodium selenite (30 nM, see Bottenstein and Sato, 1979, Proc. Natl. Acad. Sci. U.S.A. 76:514-517). Viable cell count may then be obtained by hemocytometer counting in the presence of trypan blue. The motor neuron enriched cell suspension may then be plated at a density of about 100,000 cells/cm² in tissue culture wells (preferably 6 mm) precoated with poly L-ornithine (e.g. 10 µg/ml) and laminin (e.g. 10 µg/ml). Neurotrophic factors may then be added. For example, in specific embodiments, BDNF may be added to achieve a final concentration of between about 0.01 and 100 ng/ml, and preferably about 50 ng/ml, or NT-3 may be added to achieve a final concentration of between about 0.01 and 100 ng/ml, and preferably about 50 ng/ml, or BDNF at the concentrations listed supra may be added in conjunction with CNTF at a concentration of at least about 1 ng/ml and preferably about 10 ng/ml. The motor neuron cultures may then be maintained in serum-free defined medium at 37°C in a 95% air/5% CO₂ atmosphere at nearly 100% relative humidity.

### 5.4. DIAGNOSTIC APPLICATIONS

The present invention also provides for a method of diagnosing a motor neuron disorder in a patient comprising comparing the level of neurotrophic factor that is a member of the BDNF/NT-3/NGF family in a sample from the patient with an analogous sample from a normal individual and thereby detecting an aberrancy in the level of the neurotrophic factor in the patient which positively correlates with the presence of the motor neuron disorder. Motoneuron disorders that may be diagnosed in this manner include but are not limited to those listed in Section 5.2., supra.

The neurotrophic factor level may be detected by any method known in the art including, but not limited to, enzyme-linked immunoadsorbant "sandwich" assay or any other assay that utilizes anti-neurotrophic factor antibody, including Western blot analysis and in situ hybridization, bioactivity studies, Northern blot analysis, etc.

Patient samples may be derived from any appropriate tissue or fluid, including but not limited to, nerve or brain tissue or cerebrospinal fluid or blood.

An aberrancy in neurotrophic factor level may be defined as a statistically significant difference in the level of neurotrophic factor in the patient compared to a normal control. The aberrant level may be increased or decreased relative to normal levels, and/or may exist throughout the patient or only in a localized area. Patients exhibiting a decrease in neurotrophic factor may particularly benefit from administration of the factor (see section 5.2, supra). Patients exhibiting an increase in neurotrophic factor may be expressing abnormal factor or may suffer from an effective paucity of factor receptor. Patients with fewer, or abnormal, factor receptors may be identified by testing cells of such patients for normal neurotrophic factor receptor, for example by comparing ability of such cells to bind detectably labeled neurotrophic factor to the binding capacity of normal cells. Patients with fewer receptors or abnormal receptors may or may not benefit from supplemental neurotrophic factor therapy, and may particularly benefit from treatment with neurotrophic factor agonist analogues.

In addition, the discovery that BDNF or NT-3 appears to be retrogradely transported by motor neurons provides for a method of diagnosing a BDNF or NT-3 related motor neuron disorder comprising injecting detectably labeled BDNF or NT-3 in a manner such that the labeled BDNF enters a motor nerve and determining whether the labeled BDNF or NT-3 is retrogradely transported, in which a failure to be retrogradely transported correlates with dysfunction of motor neurons. This method may be applied in the diagnosis of disorders including but not limited to amyotrophic lateral sclerosis, progressive spinal muscular atrophy, infantile muscular atrophy, poliomyelitis, post-polio syndrome, Charcot-Marie Tooth disease, and nerve trauma or injury.

### 5.5. ASSAY SYSTEMS

The present invention provides for assay systems that may be used to identify compounds that may be used in the treatment of motor neuron disorders. Such assay systems evaluate a test agent for the ability to block BDNF or NT-3 binding to motor neurons. Accordingly, the present invention provides a method for assaying a test agent for the ability to promote the survival, growth, or differentiation of motor neurons comprising exposing motor neurons in culture to detectably labeled BDNF or NT-3 (or any suitable member of the BDNF/NT-3/NGF family) under conditions such that the labeled BDNF or NT-3 is capable of binding to the motor neurons and, at the same time exposing the motor neurons to a test agent, in which inhibition of binding of labeled BDNF or NT-3 by the test agent indicates that the test agent may be used in the treatment of motor neuron disorders. A test agent that tests positive in such an assay system may desirably be further evaluated in vitro or in vivo for the ability to promote the survival, growth, or differentiation of motor neurons in culture. A test agent that tests negative may in some cases be an antagonist of BDNF or NT-3-like activity.

BDNF or NT-3 may be detectably labeled by any method, including by the incorporation of radiolabelled amino acids, and by the addition of a detectable "tag" to the neurotrophic factor molecule. Such a tag may be fluorescent, may have enzymatic activity, may be an antibody that binds to the neurotrophic factor, or may be antigenic, and capable of binding to antibody (e.g. a portion of the myc antigen that is capable of binding to anti-myc antibody).

Test agents that may be used according to the invention include, but are not limited to, neurotrophic factors that are members of the BDNF/NT-3/NGF family, or fragments or derivatives thereof, other peptide compounds, peptide derivatives, and non-peptide compounds.

In a specific, non-limiting embodiment of the invention offered as an example, radiolabelled BDNF together with unlabeled test agent is incubated with motor neurons in culture under conditions that allow BDNF binding. As a control, an identical motor neuron culture is exposed to radiolabelled BDNF in the absence of test agent. If the test agent is capable of binding to the BDNF receptor, it may competitively inhibit labeled BDNF binding. When the cells are then washed to remove unbound BDNF, the culture containing test agent may be expected to contain less radioactivity than the control culture.

### 5.6. ANIMAL MODEL SYSTEMS

The present invention further provides for animal model systems for motor neuron disorders comprising animals that have systemic or generalized depletion of BDNF or NT-3. Generalized depletion of BDNF or NT-3 may be achieved by producing an animal that expresses antibody directed toward BDNF or NT-3; such animals may be produced by immunizing the animal with BDNF or NT-3 from another species or that has been chemically modified to become more immunogenic. Local depletion may be achieved by introducing anti-BDNF or anti-NT-3 antibody locally, e.g. by a hybridoma implant in the vicinity of the precentral gyrus. Furthermore, transgenic animals may be produced in which the endogenous BDNF or NT-3 gene has been "knocked out" by homologous recombination.

### 6. EXAMPLE: BDNF INCREASED CHOLINE ACETYLTRANSFERASE ACTIVITY IN VENTRAL SPINAL CORD MOTOR NEURON CULTURES

### 6.1. MATERIALS AND METHODS

### 6.1.1. EXPERIMENTAL ANIMALS

Embryos (E14) from Sprague-Dawley rats (HSD or Zivic-Miller) were used for all experiments. Pregnant rats were sacrificed by carbon dioxide asphyxiation, and embryos were rapidly removed and placed in icecold medium for further dissection.

### 6.1.2. TISSUE CULTURE TECHNIQUES

Spinal cords were removed aseptically from rat embryos at 14 days gestation. The spinal cord was severed caudal to the bulb (at the level of the first dorsal root ganglion), and freed of sensory ganglia and adhering meninges. The cord was then subdivided into ventral and mediodorsal segments for separate cultures. The ventral spinal cord tissues were diced into small pieces and incubated in 0.1% trypsin (GIBCO) and 0.01% deoxyribonuclease type 1 (Sigma) in calcium-magnesium free phosphate buffered saline (PBS) at 37°C for 20 minutes. Trypsin solution was then removed, and the cells were rinsed and placed in medium consisting of 45% Eagle's minimum essential medium (MEM), 45% Ham's nutrient mixture F12 (F12), 5% heat inactivated fetal calf serum (GIBCO), 5% heat inactivated horse serum (GIBCO), glutamine (2 mM), penicillin G (0.5 U/ml), and streptomycin (0.5 µg/ml). The tissue was then mechanically dissociated by gentle trituration through a Pasteur pipet, and the supernatants were pooled and filtered through a nylon filter (Nitex, Tetko; 40 µm). The filtered cell suspensions were then subjected to a fractionation procedure as set forth in section 6.1.3, infra.

### 6.1.3. FRACTIONATION OF VENTRAL SPINAL CORD CELLS BY METRIZAMIDE DENSITY GRADIENT

This fractionation protocol was a modification of the method described by Schnaar and Schaffner (1981, J. Neurosci. 1:204-217). All steps were carried out at 4°C. Motrizamide was dissolved in F12:MEM (1:1) medium, and a discontinuous gradient was established which consisted of a 18% metrizamide cushion (0.5 ml), 3 ml of 17% metrizamide, 3 ml of 12% metrizamide, and 3 ml of 8% metrizamide. The filtered ventral spinal cord cell suspension (2.5 ml) obtained as described above was layered over the step gradient, the tube was centrifuged at 2500 g for 15 minutes using a swing-out rotor (Sorvall HB4). Centrifugations resulted in three layers of cells: fraction I (at 0-8% interface), fraction II (at 8-12% interface), and fraction III (at 12-17% interface). Fraction I, enriched with motor neurons, at the 0-8% interface was removed in a small volume (about 1 ml), rinsed twice with serum-free defined medium consisting of 50% F12 and 50% MEM, supplemented with glutamine (2 mM), insulin (5 µg/ml), transferrin (100 µg/ml), progesterone (20 nM), putrescine (100 µM), and sodium selenite (30 nM) (Bottenstein and Sato, 1979, Proc. Natl. Acad. Sci. U.S.A. 76:514-517). Viable cell count was obtained by hemocytometer counting in the presence of trypan blue. Cell suspension from fraction I (enriched with motor neurons) was then plated at a density of 100,000 cells/cm² in 6 mm wells precoated with poly-L-ornithine (Sigma; 10 µg/ml) and laminin (GIBCO; 10 µg/ml). Treatments with growth factors (CNTF and BDNF) were given on the day of plating. Cultures were maintained in serum-free defined medium at 37°C in 95% air/5% CO₂ atmosphere at nearly 100% relative humidity. On day 2 (48 hours), cells were harvested for measurements of choline acetyltransferase (CAT; Fonnum, 1975, J. Neurochem. 24:407-409) and see section 7.1.5, infra.

### 6.1.4. BRAIN DERIVED NEUROTROPHIC FACTOR (BDNF)

BDNF was produced in CHO cells and purified from CHO cell conditioned media to homogeneity as assessed by silver-stained polyacrylamide gels and amino acid sequence analysis (see Section 8, infra).

### 6.1.5. CILIARY NEUROTROPHIC FACTOR (CNTF)

The CNTF used in all experiments was recombinant rat CNTF expressed in E. coli and purified as described in Masiakowski et al. WO 91/04316.

### 6.1.6. OTHER FACTORS

NGF (2.5S) used in these studies was purified from mouse salivary glands according to protocols described by Mobley et al., 1976, Biochemistry 15:5543-5551. Basic FGF was obtained from Collaborative Research.

### 6.2. RESULTS

### 6.2.1. EFFECTS OF BDNF ON CHOLINE ACETYLTRANSFERASE ACTIVITY

Treatment of motor neuron enriched cultures with BDNF resulted in a 4-5-fold maximal stimulation in CAT activity after 48 hours as compared to untreated controls. The increase in CAT activity appeared to be BDNF dose dependent with maximal response achieved at approximately 10 ng/ml, as shown in Figure 1.

### 6.2.2. EFFECTS OF CO-ADDITION OF BDNF AND CNTF ON CHOLINE ACETYLTRANSFERASE ACTIVITY

Since ciliary neurotrophic factor (CNTF) has been shown to promote rat motor neuron survival in vitro (Wong et al., 1990, Soc. Neurosci. Abst.), we sought to determine whether the increase in CAT activity by these two factors (BDNF and CNTF) is additive. Co-treatment with BDNF (50 ng/ml) and CNTF (50 ng/ml) which represent saturating concentrations resulted in a more than additive increase in CAT activity in cultured motor neurons (Fig. 2), suggesting that these two factors act synergistically and have different regulatory pathways. The maximal added effect achieved by coaddition of BDNF and CNTF was about 15-fold higher than control levels. Neither NGF nor bFGF were found to have a significant affect on CAT activity compared with control cultures (Figure 2B)

Degeneration and death of motor neurons in the ventral horn of the spinal cord is a major aspect of the pathophysiologic process in amyotrophic lateral sclerosis (ALS; Lou Gehrig's disease), spinal cord injury, and other motor neuron diseases. These results suggest that BDNF alone or in combination with CNTF may be used in treating these diseases to promote cholinergic expression.

### 7. EXAMPLE: NT-3 TREATMENT INCREASED CHOLINE ACETYLTRANSFERASE ACTIVITY IN SPINAL CORD MOTOR NEURON CULTURES

### 7.1. MATERIALS AND METHODS

### 7.1.1. EXPERIMENTAL ANIMALS

Embryos (stage E14) from Sprague-Dawley rats (HSD or Zivic-Miller) were used for all experiments. Pregnant rats were sacrificed by carbon dioxide asphyxiation,and embryos were rapidly removed and placed in ice-cold medium for further dissection.

### 7.1.2. TISSUE CULTURE TECHNIQUES

Spinal cords were removed aseptically from rat embryos at 14 days gestation and prepared as set forth in section 6.1.2., supra.

### 7.1.3. FRACTIONATION OF VENTRAL SPINAL CORD CELLS BY METRIZAMIDE DENSITY GRADIENT

The fractionation protocol was as set forth in section 6.1.3., supra, except that cells were treated with NT-3 on the day of plating. As shown in Figure 3, NT-3 treatment resulted in a 3-4-fold maximal stimulation in CAT activity-in motor neurons, compared to untreated controls. The increase in CAT activity was dose dependent, with maximal response achieved at about 1 ng/ml.

### 7.1.4. NEUROTROPHIN-3 (NT-3)

NT-3 was produced in CHO cells and purified from CHO cell conditioned media to homogeneity as described for BDNF in Section 8, infra, as assessed by silverstained polyacrylamide gels and amino acid sequence analysis.

### 7.1.5. CHOLINE ACETYLTRANSFERASE ASSAY

Cultures were harvested by lysing the cells in a 20 mM Tris-HCl (pH 8.6) solution containing 0.1% Triton X-100. Two microliters of the cell lysate were removed and assayed for CAT activity according to the micro-Fonnum procedure (Fonnum, 1975, J. Neurochem. 24:407-409). The final substrate composition consisted of 0.2 mM [1-¹⁴C] Acetyl-CoA (NEN, 54.4 mCi/mmol), 300 mM NaCl, 8 mM choline bromide, 20 mM EDTA, and 0.1 mM neostigmine in 50 mM NaH₂PO₄ (pH 7.4) buffer. At these enzyme and substrate concentrations, the enzymatic reaction was linear for 90-120 minutes. The specificity of the induction for CAT was tested by the addition of a specific inhibitor of CAT activity, N-hydroxyethyl-4-(1-napthylvinyl) pyridium (HNP), during the assay (White and Cavallito, 1970, J. Neurochem. 17:1579-1589).

### 7.2. RESULTS

As discussed supra, degeneration and death of motor neurons in the ventral horn of the spinal cord is a major aspect of the pathophysiologic process in amyotrophic lateral sclerosis (ALS; Lou Gehrig's disease), spinal cord injury, and other motor neuron diseases. The instant results, which show increased CAT activity in NT-3 treated motor neurons, suggest that NT-3 may be used in treating these diseases to promote cholinergic expression.

### 8. EXAMPLE: PROCEDURE FOR THE PURIFICATION OF RECOMBINANT BRAIN DERIVED GROWTH FACTOR FORM CONDITIONED MEDIUM OF CHINESE HAMSTER OVARY CELLS

### 8.1. MATERIALS AND METHODS

Recombinant BDNF was isolated from CHO cell-conditioned medium by a two step procedure consisting of cation exchange and gel filtration chromatography.

Conditioned medium collected from Opticell bioreactor (Charles River) seeded with CHO cells expressing BDNF was diluted with distilled water at 1.25:1 ratio and continuously loaded at 4°C onto 15 ml (1.6 cm i.d.) S-Sepharose Fast Flow (Pharmacia) column. Total volume of 10.6L (diluted) was applied in a period of five days. The column was washed with 50 ml of 20 mM MES buffer (a solution of 4-morpholinethane-sulfonic acid; pH adjusted to 5.8 with NaOH) pH 5.8, followed by 50 ml of the same buffer containing 250 mM sodium chloride followed by the same MES buffer without sodium chloride until absorbance at 280 nm reached baseline level. The column was then washed with 25 ml of 20 mM bicine buffer pH 8.5 followed by 15 ml of the same buffer containing 100 mM sodium chloride. The column was subsequently eluted with 150 ml, 100 mM to 750 mM sodium chloride gradient in 20 mM bicine buffer pH 8.5. The flow rate was 2.5 ml/min. Absorbance of the eluate was monitored at 280 nm at a sensitivity of 0.2 absorbance unit full scale. Fractions containing recombinant BDNF were identified by dorsal root ganglia (DRG) explant bioassay. The fractions with the highest specific activity corresponded to sodium chloride concentration between 500 and 750 mM. These fractions were combined (45 ml) and concentrated to 2 ml by high pressure membrane ultrafiltration using 3 KDa Omega type membrane. This membrane was selected on the basis of its low nonspecific binding of BDNF. The concentrated sample was then applied onto 120 ml (1.6 cm i.d.) Sephacryl S-100HR gel filtration column (Pharmacia). The column was packed and equilibrated with 20 mM HEPES Buffer pH 7.6 containing 400 mM NaCl. The column was eluted at 0.25 ml/min. 2 ml size fractions were collected and assayed for BDNF activity in the DRG explant bioassay. Fractions eluted between 80 and 90 ml were found to contain bioactive recombinant BDNF. These fractions were combined and analyzed by reducing SDS polyacrylamide electrophoresis, N-terminal sequencing and quantitative amino acid analysis. Gel electrophoresis (18% polyacrylamide) revealed the presence of two closely spaced bands comigrating with cytochrome C. N-terminal analysis and quantitative amino acid analysis were carried out using standard procedures.

N-terminal sequencing indicated that the isolated material was composed of full length BDNF ( about 66%) and truncated BDNF lacking the first 6 amino acid residues. Quantitative amino acid analysis of the sample revealed the protein concentration to have an approximate mean value of 0.043 mg/ml. Based on this value, the final yield of pure bioactive BDNF was 0.430 mg. DRG explant bioassay required approximately 1.2 ng/ml of this material for half saturation.

### 8.2. RESULTS: QUANTITATIVE AMINO ACID ANALYSIS OF BDNF

Quantitative amino acid analysis on the purified BDNF was performed on a Beckman 6300 amino acid analyser. Results (Table I) indicate the protein had an approximate mean concentration of 0.043 mg/ml. Figure 4 depicts the dose response curve for BDNF using standard dorsal root ganglion explant bioassay.

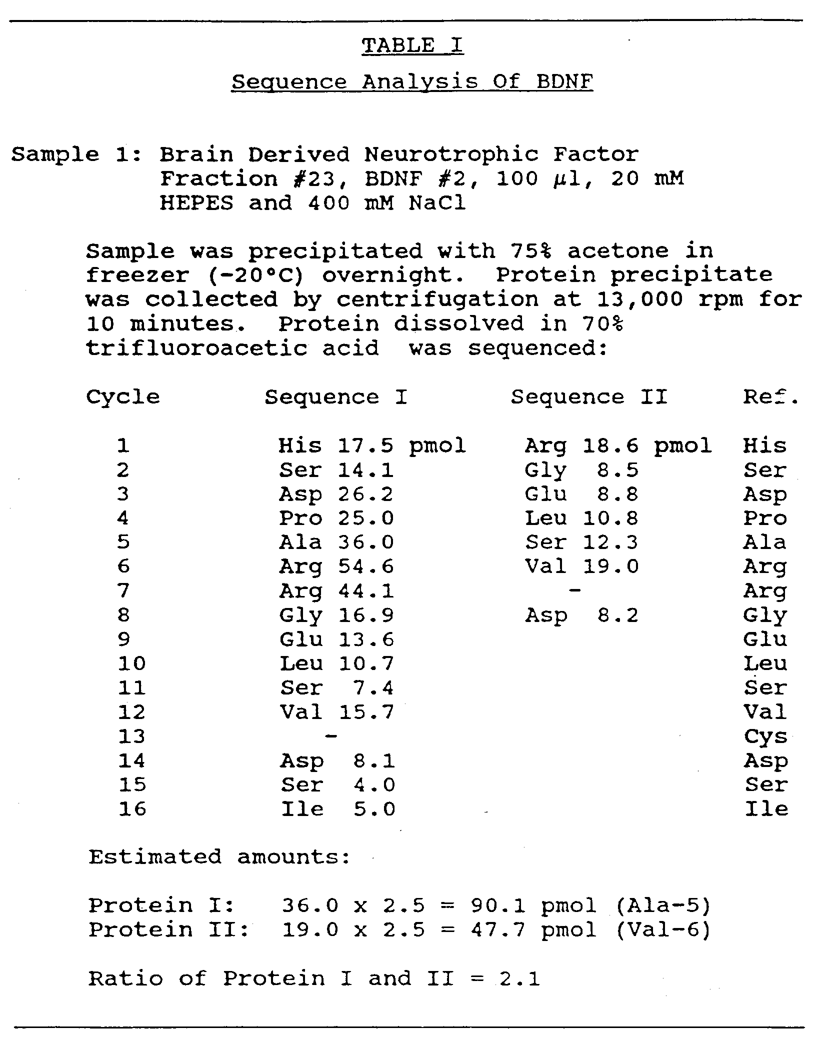

### 9. EXAMPLE: RETROGRADE TRANSPORT OF ¹²⁵I-BDNF AND ¹²⁵-NT-3 TO THE RAT VENTRAL SPINAL CORD

### 9.1. MATERIALS AND METHODS

### 9.1.1. NEUROTROPHINS

Recombinant BDNF and NT-3 were prepared from conditioned medium using the method set forth in Section 8, supra. BDNF and NT-3 were radioiodinated (Yip et at., 1983, J. Neurosci. 3:2172-2182) using the lactoperoxidase method to specific activities of 3000-7800 cpm/fmol. Free ¹²⁵I was removed prior to transport studies.

### 9.1.2. EXPERIMENTAL ANIMALS

Male Sprague-Dawley rats (250-300 g) were obtained from Zivic Miller and were used for all retrograde transport studies.

### 9.1.3. RETROGRADE TRANSPORT STUDIES

Rats were anesthetized with a mixture of chloral hydrate (4%) and sodium pentobarbital (.88%) in a volume of 3 ml/kg body weight. The right sciatic nerve was exposed and crushed for 10 seconds at a point 0.4 cm distal to the tendon of the obturator internus muscle. A crush lesion was performed for the purpose of exposing maximal numbers of receptor sites for access to BDNF. Following the crush, 2 µl of ¹²⁵I-labeled BDNF or NT-3 containing either 10 mg/ml BSA in PBS or unlabeled BDNF, NT-3 or NGF (100-fold excess) in the PBS/BSA buffer was injected into the crush site. A total of 16 ng of BDNF and 14 ng of NT-3 was injected (4x10⁶ and 2.2x10⁶ cpm, respectively). Wounds were sutured and the rats were allowed to recover for 18 hours. Rats were sacrificed and the lumbar 4 and 5 spinal cord segments were removed and sectioned into right (ipsilateral) and left (contralateral) sides. Samples were placed in 4% paraformaldehyde buffered with phosphate (pH 7.4) and counted in a gamma counter. Data are expressed as attomoles (10⁻¹⁸ moles) of ¹²⁵I-neurotrophin transported per right or left spinal cord ± S.E.M.

### 9.2. RESULTS

Table II shows that BDNF accumulated in the spinal cord of rats injected with ¹²⁵I-BDNF into the crushed sciatic nerve. Counts (300-400 counts per minutes, corresponding to about 50 attomoles) were observed on the injected side whereas near background counts accumulated on the contralateral side. The low level of counts that accumulate on the contralateral side may arise from leakage from the injection site to the site of interest through the systemic circulation. This background was routinely 15-25% of the injected side for spinal cord transport studies and these counts remained relatively constant throughout the experiment. While a 100-fold excess of BDNF blocked the transport significantly, NGF and NT-3 (>100-fold) only partially blocked the transport. Emulsion autoradiographic analysis of spinal cord tissue revealed labeling of large ventral horn neurons (in the dorso-lateral quadrant of the ventral horn) which morphologically appear to be motor neurons. Only background labeling was seen in the dorsal spinal cord.

**TABLE II**

| Retrograde Transport of ¹²⁵I-BDNF TO RAT SPINAL CORD | | |
|---|---|---|
| Injection | attomoles BDNF R | |
| | R | L |
| ¹²⁵I-BDNF + PBS | 56 ± 15 | 10 ± 4 |
| ¹²⁵I-BDNF + BDNF | 7 ± 3* | 3 ± 1 |
| ¹²⁵I-BDNF + NT-3 | 35 ± 1 | 6 ± 3 |
| ¹²⁵I-BDNF + NGF | 48 ± 5 | 8 ± 3 |
| BDNF was injected into the right crushed sciatic nerve in all experiments. Results are expressed as mean ± S.E.M. for 3-4 animals. R, right spinal cord; L, left spinal cord. | | |

| | | |
|---|---|---|
| *p < 0.05 compared to ¹²⁵I-BDNF + PBS. | | |

Table III reveals that NT-3 is also transported to the spinal cord. Again, a 100-fold excess of the homologous ligand significantly decreased transport, but NGF and BDNF were without significant effect on NT-3 transport.

**TABLE III**

| Retrograde Transport of ¹²⁵I-NT-3 TO RAT SPINAL CORD | | |
|---|---|---|
| Injection | attomoles NT-3 | |
| | R | L |
| ¹²⁵I-NT-3 + PBS | 37 ± 3 | 14 ± 9 |
| ¹²⁵I-NT-3 + NT-3 | 16 ± 7* | 11 ± 6 |
| ¹²⁵I-NT-3 + BDNF | 28 ± 5 | 10 ± 2 |
| ¹²⁵I-NT-3 + NGF | 32 ± 6 | 18 ± 4 |
| Results are expressed as mean ± S.E.M. for 3-4 animals. R, right spinal cord; L, left spinal cord. | | |

| | | |
|---|---|---|
| *p <0.05 compared to ¹²⁵I-NT-3 + PBS. | | |

### 10. EXAMPLE; BRAIN-DERIVED NEUROTROPHIC FACTOR AND NEUROTROPHIN-3 SUPPORT THE SURVIVAL OF INJURED MOTOR NEURONS IN NEWBORN RATS

The following data was supplied in a manuscript by M. Sendtner, B. Holtmann, R. Kolbeck, H. Thoenen and Y.-A. Barde of the Department of Neurochemistry and Neurobiochemistry of the Max-Planck-Institute for Psychiatry.

### 10.1. MATERIALS AND METHODS

### 10.1.1. SURGICAL AND HISTOLOGICAL PROCEDURES

The transsection of the right facial nerve in rat pups was carried out as described in Sendtner et al., 1990, Nature 345:440-441. Briefly, newborn Wistar rats were anaesthetized by hypothermia and a cut of about 3 mm length was made directly behind the right ear. The facial nerve was exposed at the foramen stylomastoideum and both roots were transsected about 1 mm distal from this position. Gel foam (Spongostan, Paesel, Frankfurt, Germany) was cut into small pieces (1X2X3mm) and soaked in 30µl of PBS containing either 5µg of BSA (Cohn Fraction V, Sigma, Deisenhofen, Germany) or trophic factor. The gel foam was inserted at the site of lesion and fixed under the ligaments which cover the nerve at this position. The skin was sutured by silk (Ethikon 3-0) and the animals returned to their mother. On postnatal day seven, the animals were killed by ether overdoses and perfused transcardially with 50 ml of 4% formaldehyde. Brainstems were dissected, postfixed for one hour, rinsed with water; and dehydrated with increasing concentrations of ethanol (70-100%). After paraffinembedding serial sections (7µm) were made from the whole brain stem with a serial section microtome (Reichert-Jung 2050 supercut). The sections were stained with cresyl violet (Sigma, Deisenhofen, Germany), and the nucleoli of the facial motoneurons in both control and lesion sides were counted under the light microscope (magnification 125x) in every fifth section as described in Sendtner et al. The counts were not corrected for split nucleoli.

### 10.1.2. PRODUCTION AND CHARACTERIZATION OF NGF, BDNF AND NT-3

Nerve growth factor (2.5 S) was isolated from submaxillary glands of adult male mice. Both recombinant mouse BDNF and NT-3 were produced in rabbit kidney cells after transfection with a recombinant vaccinia virus and purified from supernatants.

### 10.2. RESULTS

If the axons of facial motor neurons were transected or crushed, more than 80% of the lesioned cells degenerate within few days. The application of CNTF to the lesion site has been shown to increase the survival rates of these cells to nearly 100%. Under the same conditions the application of 5µg of NGF did not appear to significantly enhance the survival of motoneurons.

In contrast to the animals treated with NGF, significant survival rates could be observed if BDNF was applied to the transsected facial nerve. On average, about 50% of the lesioned motor neurons were still alive one week after lesion (Table IV). The survival rates ranged from 765 to 4580 neurons at the lesion site. In the animal where only 765 neurons could be detected, the gel foam containing trophic factor was displaced from the lesion site and this probably explains the low survival effect of BDNF seen in this single experiment.

As with BDNF, there was also an enhanced survival of motor neurons following NT-3 treatment (Table IV). However, the effects were smaller than those observed with BDNF; on average, 27 percent of the lesioned motor neurons would be rescued on the lesion side. Compared to 18 percent of motor neurons rescued by BSA, the survival-promoting effects of NT-3 are statistically significant (p<0.05).

Morphologically, in both BDNF and NT-3 treated animals, the lesioned motor neuron cell bodies were smaller and showed typical reactive changes (Fig. 5). The nuclei were displaced and chromatolysis was pronounced both in BDNF and NT-3 treated animals. However, when compared to control animals treated with BSA-gel foam, the size of the nuclei appeared greater (Fig. 5).

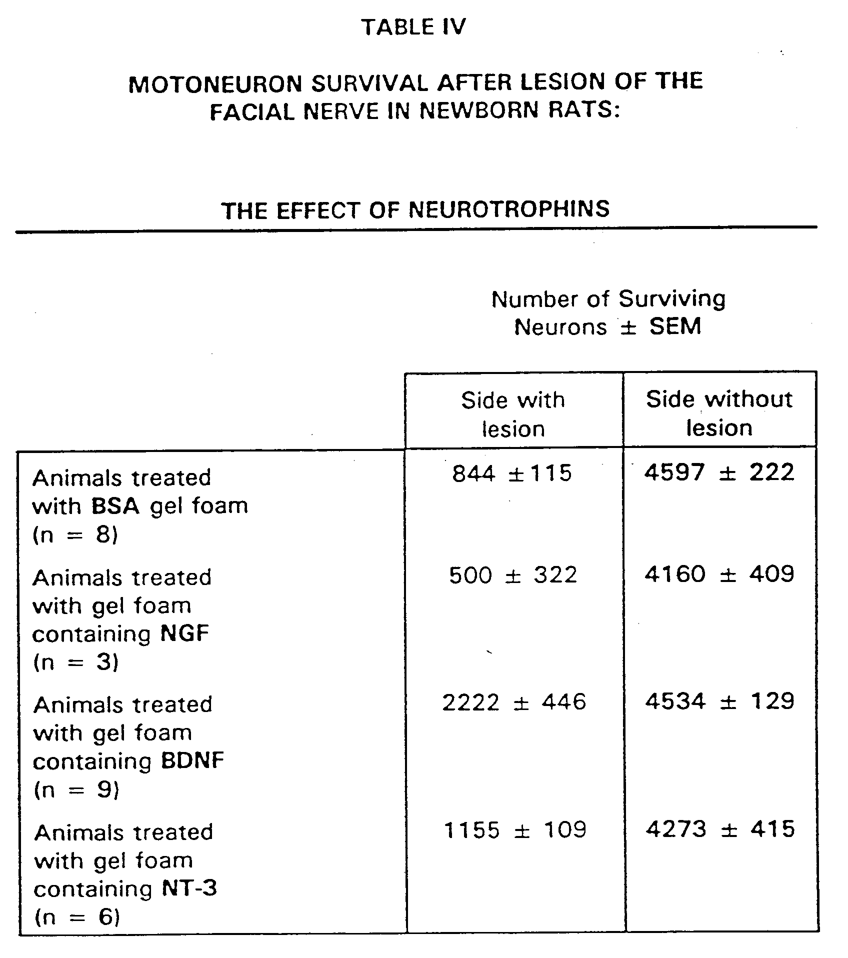

### 10. 3. DISCUSSION

In the present study we have shown that BDNF is able to support the survival of a significant number of facial motoneurons after axotomy in the newborn rat. In addition, NT-3 also seems to be active on these cells, although to a lower degree. In contrast, NGF appears not to support the survival of these cells after axotomy. These data show that members of the neurotrophin gene family are capable of affecting motoneuron survival in vivo in a similar manner to CNTF.

Various publications are cited herein, the disclosures of which are incorporated by reference in their entireties.

## Claims

1. Use, in the manufacture of a medicament for the treatment of a motor neuron disorder, of
(a) Brain-Derived Neurotrophic Factor
(BDNF); and
(b) ciliary neurotrophic factor (CNTF).

2. Use according to claim 1 wherein the motor neuron disorder is caused by amyotrophic lateral sclerosis; trauma; progressive spinal muscular atrophy; infantile or juvenile muscular atrophy; policmyelitis or post polio syndrome; or Charcot-Marie-Tooth disease.

3. A pharmaceutical composition that may be used in the treatment of a motor neuron disorder comprising:
(a) Brain-Derived Neurotrophic Factor
(BDNF); and
(b) ciliary neurotrophic factor (CNTF)
in a pharmacologically acceptable carrier.
